# EUROPEAN PATENT SPECIFICATION

(11) **EP 1 224 469 B1**
(45) Date of publication and mention of the grant of the patent: **06.04.2005**
(21) Application number: 00969693.1
(22) Date of filing: 19.10.2000
(51) Int. Cl.: G01N 33/68, C12Q 1/37, C07K 7/02, C07K 7/04

(54) **METHOD OF SCREENING FOR INHIBITORS OF ASP2**
VERFAHREN ZUM SCREENING VON INHIBITOREN VON ASP2
PROCEDE DE DEPISTAGE DES INHIBITEURS DE ASP2

(30) Priority: 21.10.1999 GB 9924957
(43) Date of publication of application: 24.07.2002
(73) Proprietor: SmithKline Beecham plc, Brentford, Middlesex TW8 9GS (GB); SmithKline Beecham Corporation, Philadelphia, PA 19101 (US)
(72) Inventor: CHRISTIE, Gary SmithKline Beecham Pharmaceuticals, Harlow Essex CM19 5AW (GB); HUSSAIN, Ishrut SmithKline Beecham Pharmaceuticals, Harlow Essex CM19 5AW (GB); POWELL, David SmithKline Beecham Pharmaceuticals, Harlow Essex CM19 5AW (GB)
(74) Representative: Valentine, Jill Barbara
(86) International application number: PCT/GB2000/004039
(87) International publication number: WO 2001/029563

(56) References cited:
- EP-A- 0 855 444
- WO-A-01/00663
- WO-A-95/13084
- WO-A-96/40885
- WO-A-98/15828
- SAHASRABUDHE, SUDHIR R. ET AL: "Enzymic generation of the amino terminus of the.beta.-amyloid peptide" J. BIOL. CHEM. (1993), 268(22), 16699-705 , XP000573976
- ERMOLIEFF, JACQUES ET AL: "Proteolytic Activation of Recombinant Pro-memapsin 2 (Pro-.beta.-secretase) Studied with New Fluorogenic Substrates" BIOCHEMISTRY (2000), 39(40), 12450-12456 , XP002162928
- A K GHOSH, D SHIN, D DOWNS, G KOELSCH, X LIN, J ERMOLIEFF, J TANG: "Design of Potent Inhibitors for Human Brain Memapsin 2 (beta-Secretase)" JOURNAL OF THE AMERICAN CHEMICAL SOCIETY, vol. 122, March 2000 (2000-03), pages 3522-3523, XP002162929 cited in the application
- HONG, LIN ET AL: "Structure of the protease domain of memapsin 2 (.beta.-secretase) complexed with inhibitor" SCIENCE (WASHINGTON, D. C.) (2000), 290(5489), 150-153 , XP002162930
- SINHA SUKANTO ET AL: "Purification and cloning of amyloid precursor protein beta-secretase from human brain." NATURE (LONDON), vol. 402, no. 6761, 2 December 1999 (1999-12-02), pages 537-540, XP002162931 ISSN: 0028-0836 cited in the application
- HUSSAIN ISHRUT ET AL: "Identification of a novel aspartic protease (Asp 2) as beta-secretase." MOLECULAR AND CELLULAR NEUROSCIENCE, vol. 14, no. 6, December 1999 (1999-12), pages 419-427, XP000908710 ISSN: 1044-7431 cited in the application

## Description

The present invention relates to an assay used in identifying compounds which are potentially useful in therapy. The present invention also relates to the use of modulators of polypeptide cleavage in therapy.

β amyloid (Aβ) protein-related diseases are a heterogeneous class of disorders characterised by the deposition within the brain of insoluble deposits of the Aβ protein (Roberts G W, Leigh P N and Weinberger D. Neuropsychiatric Disorders. Gower Medical press. London 1993). The eventual consequence of substantial numbers of Aβ deposits is the emergence of a clinical syndrome of cognitive decline and increasing dementia. Such deposits have been shown to be present in a number of dementing syndromes and these include Alzheimer's disease, cortical Lewy body disease, Parkinson's disease and the Alzheimer-type disease in patients with Down's syndrome. In addition Aβ deposits are present in the brains of patients with vascular and cerebrovascular disease (Adams J H and Duchen L W (eds) Greenfields Neuropathology 5th Ed. Edward Arnold, London 1992) and these latter conditions can predispose or contribute to the above diseases.

Alzheimer's disease (AD) is a progressive degenerative disease of the central nervous system characterized clinically by dementia and neuropathologically by the presence of numerous senile plaques and neurofibrillary tangles. AD is typically a late onset disease of the elderly. However, a small number of pedigrees have been described wherein an early onset form of the disease is inherited as an autosomal dominant with age dependent penetrance. Most commonly, the age of onset of the disease is below 60 years old. Genetic factors have been implicated in both early and late onset AD.

Production and deposition of the 39-43 residue amyloid-β protein (Aβ , Glenner, G.G. & Wong, C.W. *Biochem. Biophys. Res. Commun.* 120, 885-890 (1984)) in the brain is an invariant neuropathological feature of AD. Aβ is produced by excision from the type 1 integral membrane glycoprotein Amyloid Precursor Protein (APP) by the sequential actions of first β- then γ-secretases (Selkoe, D.J. *Annu. Rev. Cell. Biol*. 10, 373-403 (1994)).

The APP can be cleaved in cells by α- or β- secretases, resulting in the release of soluble N-terminal fragments of the protein (sAPPα and sAPPβ). The resulting membrane anchored C-terminal fragments (CTFα and CTFβ) are substrates for γ-secretase; cleavage of CTFα giving rise to the 3kDa peptide p3 and CTFβ giving rise to the Aβ peptide. The β-secretase cleavage event has been shown to occur within several intracellular organelles, including the rough endoplasmic reticulum and the trans-Golgi network (Hartmann. *et al*., *Nature Medicine.* 3, 1016-1020 (1997), Cook, D.G. *et al., Nature Medicine.* 3, 1021-1023 (1997), Wild-Bode, C. *et al., J. Biol. Chem.* 272, 16085-16088 (1997)). Aβ is generated at a slow rate intracellularly prior to its secretion and an intraneuronal pool of Aβ has been reported that accumulates with time in the cultured cells (Skovronsky, D.M., Doms, R.W. & Lee, V.M.-Y. *J. Biol. Chem.* 141, 1031-1039 (1998)).

The secretases involved in the processing of APP have not been identified, but inhibitor studies have suggested that α-secretase is a metalloproteinase (Parvathy, S., Hussain, I., Karran, E.H., Turner, A.J. & Hooper, N.M. *Biochemistry* 37, 1680-1685 (1998)). A number of candidate β-secretases have been proposed and discounted such as the proteasome (Ishiura, S., Tsukahara, T., Tabira, T. & Sugita, H. *FEBS Lett.* 257, 388-392 (1989)), the metalloproteinase thimet (McDermott, J.R., Biggins, J.A. & Gibson, A.M. *Biochem. Biophys. Res. Commun.* 185, 746-752 (1992)), several chymotrypsin like serine proteinases (Nelson, R.B., Siman, R., Iqbal, M.A. & Potter, H. *J*. *Neurochem* 61, 567-577 (1993), Sahasrabudhe, S.R. *et al., J. Biol. Chem.* 268, 16699-16705 (1993), Savage, M.J. *et al*., *Neuroscience* 60, 607-619 (1994)), the metalloproteinases MP78 (Thompson, A., Grueninger-Leitch, F., Huber, G. & Malherde P. *Brain Res.* 48, 206-214 (1997)) and MP100 (Huber, G. *et al., J. Neurochem.* 72, 1215-1223 (1999) and cathepsin D (Ladror, U.S., Snyder, S.W., Wang, G.T., Holzman, T.F. & Krafft, G.A. *J*. *Biol. Chem.* 269, 18422-18428 (1994)). Recently it has been reported that presenilin-1 is either a unique diaspartyl cofactor for γ-secretase or is γ-secretase itself (Wolfe, M.S. *et al., Nature.* 398, 513-517 (1999)). WO96/40085 proposes a candidate β-secretase isolated from human brain tissue and human 293 cells having an apparent molecular weight in the range from 260kDa to 300kDa when measured by gel exclusion chromatography.

Asp 2 (also known as endocrepsin 2) is a transmembrane aspartyl proteinase that shows high levels of expression in brain and pancreas (EP0855444). The residue at position 130 has subsequently been shown to be Valine, and not Glutamic acid as previously described in EP0855444 (Hussain, I. *et al, Molec.Cell.Neuosci*, 14, 419-427, 1999). The proteinase has a molecular weight of 60-65 kDa when measured by gel electrophoresis after transient transfection into cells. The mature form of Asp 2 begins at residue Glu 46 (Sinha S *et al, Nature* 402, 537-540, 1999). A splice variant has also been found (WO00/17369).

Ghosh et al, J. Amer. Chem. Soc. 2000 122, 3522-3523 disclose inhibitors for memapsin 2 (Asp 2) including OM99-2 (Glu-Val-Asn-Leu*Ala-Ala-Glu-Phe where * designates the hydroxyethylene transition state isostere.

The present invention is based on the finding that Asp 2 can function in the β-secretase cleavage pathway of APP. The proteinase has many of the expected characteristics of β-secretase, in that it is present in the brain, including AD brain, and is also found in cell lines known to produce Aβ and co-localises with APP in the Golgi/endoplasmic reticulum of cells stably expressing the 751 amino acid isoform of APP. Additionally, Asp 2 has been found to be a type I integral membrane protein with the catalytic domain residing in the lumen of membranous organelles. Transfection of Asp 2 into APP expressing cells results in an increase in the β-secretase activity in cells, such that more sAPPβ is secreted into the medium and there is an accumulation of the β-secretase derived C-terminal fragment. Mutation of either of the proposed catalytic aspartyl residues in Asp 2 abrogates the production of these fragments which are charateristic of the β-secretase cleavage of APP. These findings suggests that inhibition of the proteolytic activity of Asp 2 has potential value as a therapy for the treatment of β amyloid protein-related disease including Alzheimer's Disease.

The present invention therefore provides a method of screening compounds to identify those compounds which inhibit the Asp 2 mediated cleavage of a polypeptide or protein substrate, the method comprising: providing a reaction system comprising Asp 2 and substrate; and measuring the extent of cleavage of the substrate in the presence of test compound as compared with the extent of cleavage in the absence of test compound. The invention also relates to compounds identified thereby and their use in therapy including the treatment or prophylaxis of β amyloid protein-related disease including AD.

The substrate is a protein or peptide that is capable of being hydrolysed by the Asp 2 enzyme. This may be a non-specific protein substrate, such examples being casein, haemoglobin, insulin B-chain, cytochrome C, etc. It may also be recombinant full length or truncated amyloid precursor protein. Substrates may also be peptides, which are often synthetic fragments of larger proteins. For example, a peptide spanning the beta-secretase cleavage site of APP may serve as a convenient substrate, this peptide possibly including the wild-type beta-site sequence (from P6 to P5', terminology as described by Berger, A, & Schecter, I. *Philos. Trans. R. Soc. Lond. [Biol.]* 257, 249-264, (1970)) Ile-Ser-Glu-Val-Lys-Met-Asp-Ala-Glu-Phe-Arg (SEQ ID NO.1) or the Swedish variant sequence (from P6 to P5') Ile-Ser-Glu-Val-Asn-Leu-Asp-Ala-Glu-Phe-Arg (SEQ ID NO.2) Smaller or longer peptides of the wild-type or Swedish variant sequences, and other variants based on these sequences, may also be used.

Larger proteins modified to encode Asp 2 substrate sequences may also serve as useful substrates for screening. For example, maltose binding protein (MBP) can be modified to encode the wild-type or Swedish variant beta-site sequences at its C-terminus to generate 
Maltose Binding Protein-Ile-Ser-Glu-Val-Lys-Met-Asp-Ala-Glu-Phe-Arg
(wild-type beta-site) (SEQ ID NO.3)
 or

Maltose Binding Protein-Ile-Ser-Glu-Val-Asn-Leu-Asp-Ala-Glu-Phe-Arg
(Swedish Variant beta-site) (SEQ ID NO.4)

These could be further modified to a encode C-terminal Q-Tag (Leu-Ser-Leu-Ser-Gln-Ser-Lys-Val-Leu-Pro-Gly-Pro (SEQ ID NO.5)) to generate 
Maltose Binding Protein-Ile-Ser-Glu-Val-Lys-Met-Asp-Ala-Glu-Phe-Arg-
Leu-Ser-Leu-Ser-Gln-Ser-Lys-Val-Leu-Pro-Gly-Pro (wild type beta-site)
(SEQ ID NO.6)
 or

Maltose Binding Protein-Ile-Ser-Glu-Val-Asn-Leu-Asp-Ala-Glu-Phe-Arg-
Leu-Ser-Leu-Ser-Gln-Ser-Lys-Val-Leu-Pro-Gly-Pro (Swedish Variant
beta-site) (SEQ ID NO.7)

These proteins can be fluorescently labelled via the Q-Tag to generate substrates suitable for use in various fluorescent formats.

The assay may be carried out in cell free or cell based reaction system using conventional assay formats such as those described in WO96/40885.

The Asp 2 enzyme used in the present invention includes isoforms including splice variants.

A cell based assay will comprise a host cell cotransfected with expression vectors containing DNA encoding Asp 2 and the substrate.

The presence of the cleavage products may be detected by assaying the protein content of the host cell by using polyclonal or monoclonal antibodies raised against substrate fragments. Any suitable configuration of immunoassay may be employed, for example a Western blot assay or an ELISA.

A cell free assay will comprise purified recombinant Asp 2, optionally as an Fc fusion, and substrate in a suitable reaction buffer. The enzyme is preferably predominantly in the mature form.

The enzyme may be presented as an Fc fusion in order to facilitate purification using protein A affinity chromatography. Suitably the Fc region is derived from human IgG.

In a cell free assay, the cleavage products may be detected by immunoassay as described above. Alternatively, the peptide substrate may contain a pair of marker groups, which straddle the cleavage site. Cleavage separates the markers and this change can be detected using techniques which reflect colocalisation of these markers. Detection may depend on an optical interaction between the two markers, or more generally, signal generation may be dependent upon their colocalisation in the substrate. Techniques based on optical interactions include fluorescent energy transfer (FQ), as described by Forster theory, and luminescence energy transfer (Selvin and Hearst, *Proc. Nat. Acad*. *Sci. USA*, 1994, 91, 10024). Assays based on changes in translational or rotational diffusion include fluorescence correlation spectroscopy (FCS) (Eigen and Rigler, *Proc. Nat. Acad. Sci. USA,* 1994, 91, 5740) and fluorescence polarisation (FP) (Levine *et al., Anal Biochem.,* 1997, 247, 83). Radioactivity based assays include scintillation proximity assays and nitrocellulose filtration techniques. Surface adsorption techniques include immunoassays with either absorbance, fluorescence, chemiluminescence or time resolved fluorescence (TRF) detection (Wallac OY, Finland).

Thus, cleavage of the substrate directly or indirectly results in the modulation of a signal, for example a radioactive, luminescent or fluorescent signal.

One marker group carries the signal generator or is capable of binding to a separate reporter system . The reporter system itself may carry the signal generator or may bind to a further signalling moiety. The other marker group performs the modulator function or is capable of binding a molecule such that the bound complex itself performs the modulator function.

Examples of signal generator groups include radioactive, luminescent (triplet state emission) and fluorescent (singlet state emission) labels.

Examples of marker groups capable of binding a separate reporter system include ligands for antibodies, enzymes and receptors. The antibody, enzyme or receptor reporter system is itself labelled or is capable of participating in an immunoassay. A suitable example of such a ligand is dinitrophenol which can be captured with anti-dinitrophenol antibody followed by a suitable immunoassay.

Examples of modulator groups include moieties which modulate the optical properties of the fluorescent or luminescent labels or of the substrate as a whole when both said label and moiety are attached covalently or non-covalently to the substrate. Upon proteolytic cleavage of the substrate, the optical properties of the label, or of the molecular entity as a whole, are modulated such that proteolytic activity can be monitored spectroscopically.

Examples of groups capable of effecting the modulator function or of binding a molecule to form a modulator complex include ligands for proteins, such as biotin ligands capable of binding streptavidin or avidin, or haptens for antibodies either in solution or immobilised. Biotin ligands include biotins optionally derivatised with suitable linker groups such as aminohexanoyl.

Examples of signal generator groups and other modulator groups which modulate the optical properties of a fluorescent signal generator include fluorophores (molecular families that exhibit absorption and fluorescence spectral ranges), such as coumarins, xanthenes (including rhodamines, models and fluoresceins), fluorescamine derivatives, napthalenes, pyrenes, quinolines, resorufins, difluoroboradiazaindacenes, acridines, pyridyloxazoles, isoindols, dansyls, dabcyls, dabsyls, benzofuranyls, phthalimides, naphthalimides, and phthalic hydrazides (including luminol and isoluminol).

Examples of suitable label/modulator pairs include conventional fluorescence energy transfer or quenched fluorescence (FQ) donor/acceptor systems such as rhodamine/rhodamine, in particular rhodamine green/tetramethylyrhodamine, fluoresceins/rhodamines, fluoresceins/coumarins, 5-dimethylamino-1-naphthalenesulfonyl (DANSYL)/4-(4'-dimethylaminophenylazo)benzoic acid (DABCYL) or 5-(2-aminoethyl)aminonaphthalene-1-sulfonic acid (EDANS)/DABCYL whereby the absorption spectrum of the acceptor overlaps the emission spectrum of the donor such that changes in energy transfer are observed upon cleavage of the peptide according to Forster (1948) theory.

For detection using FQ, the marker group pair is preferably chosen from amino acids bearingthe combinations EDANS/DABCYL and fluoresceins/rhodamines. Most preferably the pair is 5-(and/or 6)-carboxyfluorescein with 5-(and/or 6)-tetramethylrhodamine (TAMRA).

Other examples of labels include lanthanide ions (typically terbium and europium) as luminescent donors for lanthanide resonance energy transfer to fluorescent or chromophoric acceptors (e.g exemplified by homogeneous time-resolved fluorescence (HTRF) technology or lanthanide chelate excitation (LANCE) technology). Spin-coupled quenching of a lanthanide donor is also possible with a nitroxide radical acceptor, typically a piperidinyloxy or pyrrolidinyloxy radical. (See M.V. Rogers (1997) *DDT* 2(4) 156).

Where the modulator group is a moiety which modulates the optical properties of the substrate as a whole upon proteolytic cleavage of the substrate, examples of suitable label/modulator pairs include a fluorescent label and a ligand for a protein, such as a biotin ligand capable of binding streptavidin or avidin. Changes in the rotational diffusion of the peptide resulting from cleavage can be monitored by observing changes in fluorescence polarisation (FP). Alternatively fluorescence correlation spectroscopy (FCS) can be used to mointor changes in translational diffusion.

Thus, for detection using FP or FCS, the marker groups are chosen from amino acids bearing a fluorophore, as defined above, combined with an amino acid bearing a protein ligand such as biotin derivatives, or haptens such as difluoroboradiazaindacenes, dansyls, dinitrophenols, fluorosceins, rhodamines and naphthalimides. The marker group pair is preferably a fluorophore/biotin ligand combination. Most preferably the pair is 5- (and/or 6)-carboxyfluorescein with aminohexanoate linked biotin (biotin-X).

In a preferred aspect the the marker group pair provides a fluorescence-quench (FQ), a fluorescence-polarisation (FP) or a fluorescence correlation spectroscopy (FCS) assay.

Marker groups are preferably fluorophore and protein ligand derivatives of amino acids with side chains readily capable of chemical modification such as lysine, ornithine, cysteine, homocysteine, serine, homoserine and tyrosine.

In a preferred aspect marker groups are or comprise modified lysine groups of the formula: 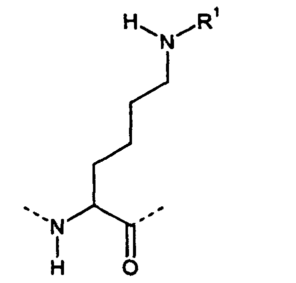 wherein R¹ is selected from suitable marker groups that are attached directly, or indirectly *via* a linking moeity, to the lysine, such that the marker groups together form a marker group pair as above described.

The substrates may be prepared by any appropriate conventional method of peptide synthesis. This includes strategies based on, for example, the Fmoc- and Boc-versions of solid phase synthesis and including sequential and fragment variations, or combinations thereof, for the chain assembly. Also are included the many different approaches for the chemical synthesis of peptides by the solution method, again utilising sequential or fragment assemblies, or combinations thereof. Other synthetic approaches can also be considered, such as those based on enzymatic coupling, etc. To those skilled in the art it will be realised that for the synthesis of peptides there are many variations possible, for example starting with different protecting groups, resins and linkers, coupling reagents, solvents, deblocking reagents, etc. Examples of such processes can be found in textbooks, including, for example, 'Solid Phase Synthesis by J M Stewart and J D Young', San Francisco, Freeman, 1969; 'The Chemical Synthesis of Peptides', J Jones, Clarendon Press, Oxford, 1991; 'Principles of Peptide Synthesis', M Bodanszky, Springer-Verlag, NY, NY, 1984; 'Solid Phase Peptide Synthesis', E Atherton and R C Sheppard, IRL Press, Oxford University Press, Oxford, 1989. More modem approaches are presented in the well known series of Proceedings from recent symposia, including, 'Innovations and Perspectives in Solid Phase Synthesis', Ed R Epton, and those conferences arranged by the European and American Peptide Societies and published under the title, 'Peptides'.

Introduction of the marker groups is accomplished by conventional methods, for example by the addition under basic conditions of either an activated ester (e.g. succinimidyl), a mixed anhydride (e.g. ethoxycarbonyl), an acid chloride, a maleimide, an isocyanide or an isothiocyanide derivative of the marker group to the base substrate (resin bound or in solution) in which a single lysine, ornithine, serine or homoserine residue bears an unprotected primary amine or hydroxyl in the side chain. Alternatively, the base substrate (resin bound or in solution) in which a single cysteine or homocysteine residue remains unprotected is reacted with either a primary alkyl halide or maleimide derivative of the marker/reporter group under basic conditions.

Generally the rate of cleavage in the absence of test compound will be known, as will the extent of cleavage at given time points. The assay may test for inhibition of cleavage at specified time points or of the rate of cleavage.

Substrate cleavage may be carried out either in solution or utilising a solid support.

The test compound may be pre-incubated with the protease prior to the addition of the substrate, or alternatively the substrate may be added directly. Final concentrations of protease and substrate are calculated so as to achieve a suitable rate of processing for carrying out the assay. The reaction may be stopped, for example by addition of methanol or trifluoroacetic acid, and the products analysed using any conventional system.

For example, reverse phase HPLC with UV detection can be used (see, for example, Kuo, D, *et al., Biochemistry*, 8347 (1994) and Allsop *et al., Bioorganic and Med. Chem. Lett.,* 443 (1995)). The activity of test compounds can be expressed as the %reduction in enzyme activity at given concentrations. In the HPLC asay this is calculated as the reduction in product peak area compared to the control. Where the substrate contains a marker pair, methanol or an exogenous binding protein may alternatively be used to stop the reaction and the products analysed using any conventional system appropriate to the choice of marker groups utilised.

Radioactive methods include the use of a biotin/radiolabel pair. The substrate is captured onto streptavidin coated flashplates, streptavidin-coated scintillation proximity assay beads or by conventional filtration (e.g nitrocellulose) techniques. Detection of the radiolabel may be carried out by way of scintillation counting.

Antibody-based peptide detection methods include the use of a ligand/ligand pair e.g. biotin/dinitrophenol label Following capture via one ligand e.g onto streptavidin coated plates, detection of the immobilised substrate is carried out using antibodies to the other ligand e.g antiDNP antibodies followed by an immunoassay such as enzyme linked immunosorbent assay (ELISA), dissociation enhanced time resolved fluorescence (DELFIA technology, Wallac OY), immunosorbent luminescence chemiluminescence or fluorescence detection.

Optical methods measuring changes in energy transfer can be carried out either macroscopically via total fluorescence intensity using a fluorimeter or by signal processing of photon emissions from individual fluorescence molecules via fluorescence correlation spectroscopy (FCS) using algorithms developed e.g. by Evotec Biosystems GmbH. Similar algorithms can be applied to determination of proteolysis rates using FCS via changes in the molecular brightness and particle number of dual and/or indirectly labelled peptide substrates.

Where the modulator group is a moiety which modulates the optical properties of the substrate as a whole upon proteolytic cleavage of the substrate, changes in fluorescence polarisation (FP) as a result of cleavage of e.g a dual biotinylated and fluorescent labelled peptide, either without or most preferably with the addition of e.g streptavidin or avidin, can be used to monitor protease activity. Changes in diffusion time of this fluorescently labelled peptide substrate as a result of proteolysis, either with or without the addition of streptavidin or avidin, can also be monitored by translational FCS. Fluorescence polarisation may be measured e.g. on a fluorescence polarisation platereader.

Inhibitors identifiable by the method of the invention are active site ligands which can be labelled to create reagents for use in competitive binding assays.

The present invention therefore also provides a method of screening compounds to identify those compounds which inhibit the Asp 2 mediated cleavage of a polypeptide or protein substrate, the method comprising: providing a reaction system comprising Asp 2 and labelled active site ligand; and measuring the extent of binding of labelled ligand in the presence of test compound as compared with the extent of binding of labelled ligand in the absence of test compound in order to determine binding affinity.

The invention also relates to compounds identified thereby and their use in therapy including the treatment or prophylaxis of β amyloid protein-related disease including AD.

Assay methods for the ligand are conventional and include those based on translational or rotational diffusion as described above for cleavage assays. In a preferred aspect the screening method is based on fluorescence polarisation (FP). A preferred label is a fluorophore as specified above, preferably rhodamine green.

The assay may be carried out in cell free or cell based reaction system using conventional assay formats such as described above for the cleavage assay.

Host cells are genetically engineered (transduced or transformed or transfected) with vectors which may be, for example, a cloning vector or an expression vector. The vector may be, for example, in the form of a plasmid, a cosmid, a phage, etc. The engineered host cells can be cultured in conventional nutrient media modified as appropriate for activating promoters, selecting transformants or amplifying the genes. The culture conditions, such as temperature, pH and the like, are those previously used with the host cell selected for expression, and will be apparent to the ordinarily skilled artisan.

Suitable expression vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it is replicable and viable in the host.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the *E. coli. lac* or *trp*, the phage lambda P_{L} promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in *E. coli*.

The gene can be placed under the control of a promoter, ribosome binding site (for bacterial expression) and, optionally, an operator (collectively referred to herein as "control" elements), so that the DNA sequence encoding the desired protein is transcribed into RNA in the host cell transformed by a vector containing this expression construction. The coding sequence may or may not contain a signal peptide or leader sequence. The protein sequences of the present invention can be expressed using, for example, the *E*. *coli* tac promoter or the protein A gene (spa) promoter and signal sequence. Leader sequences can be removed by the bacterial host in post-translational processing. Promoter regions can be selected from any desired gene using CAT (chloramphenicol transferase) vectors or other vectors with selectable markers. Two appropriate vectors are PKK232-8 and PCM7. Particular named bacterial promoters include lacI, lacZ, T3, T7, gpt, lambda P_{R}, P_{L} and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

In addition to control sequences, it may be desirable to add regulatory sequences which allow for regulation of the expression of the protein sequences relative to the growth of the host cell. Regulatory sequences are known to those of skill in the art, and examples include those which cause the expression of a gene to be turned on or off in response to a chemical or physical stimulus, including the presence of a regulatory compound. Other types of regulatory elements may also be present in the vector, for example, enhancer sequences.

An expression vector is constructed so that the particular coding sequence is located in the vector with the appropriate regulatory sequences, the positioning and orientation of the coding sequence with respect to the control sequences being such that the coding sequence is transcribed under the "control" of the control sequences (i.e., RNA polymerase which binds to the DNA molecule at the control sequences transcribes the coding sequence). Modification of the coding sequences may be desirable to achieve this end. For example, in some cases it may be necessary to modify the sequence so that it may be attached to the control sequences with the appropriate orientation; i.e., to maintain the reading frame. The control sequences and other regulatory sequences may be ligated to the coding sequence prior to insertion into a vector, such as the cloning vectors described above. Alternatively, the coding sequence can be cloned directly into an expression vector which already contains the control sequences and an appropriate restriction site. Modification of the coding sequences may also be performed to alter codon usage to suit the chosen host cell, for enhanced expression.

Generally, recombinant expression vectors will include origins of replication and selectable markers permitting transformation of the host cell, e.g., the ampicillin resistance gene of *E. coli* and *S. cerevisiae* TRP1 gene, and a promoter derived from a highly-expressed gene to direct transcription of a downstream structural sequence. The heterologous structural sequence is assembled in appropriate phase with translation initiation and termination sequences, and preferably, a leader sequence capable of directing secretion of translated protein into the periplasmic space or extracellular medium. Optionally, the heterologous sequence can encode a fusion protein including an N-terminal identification peptide imparting desired characteristics, e.g., stabilization or simplified purification of expressed recombinant product.

The vector containing the appropriate DNA sequence as hereinabove described, as well as an appropriate promoter or control sequence, may be employed to transform an appropriate host to permit the host to express the protein.

Examples of recombinant DNA vectors for cloning and host cells which they can transform include the bacteriophage λ (*E*. *coli*), pBR322 (*E. coli*), pACYC177 (*E. coli*), pKT230 (gram-negative bacteria), pGV 1106 (gram-negative bacteria), pLAFR1 (gram-negative bacteria), pME290 (non-*E*. *coli* gram-negative bacteria), pHV14 (*E. coli* and *Bacillus subtilis*), pBD9 (*Bacillus*), pIJ61 (*Streptomyces*), pUC6 (*Streptomyces*), YIp5 (*Saccharomyces*), a baculovirus insect cell system, , YCp 19 (*Saccharomyces*). See, generally, "DNA Cloning": Vols. I & II, Glover *et al*. ed., IRL Press Oxford (1985) (1987) and; T. Maniatis *et al*., ("Molecular Cloning" Cold Spring Harbor Laboratory (1982).

In some cases, it may be desirable to add sequences which cause the secretion of the polypeptide from the host organism, with subsequent cleavage of the secretory signal.

Yeast expression vectors are also known in the art. See, e.g., U.S. Patent Nos. 4,446,235; 4,443,539; 4,430,428; see also European Patent Applications 103,409; 100,561; 96,491. pSV2neo (as described in Southern, PJ and Berg, PJ, *Mol. Appl. Genet.* 1:327-341 (1982)) which uses the SV40 late promoter to drive expression in mammalian cells or pCDNA1neo, a vector derived from pCDNA1(Nelson, J *et al*., *Mol. Cell Biol*. 7:4125-29 (1987)) which uses the CMV promoter to drive expression. Both these latter two vectors can be employed for transient or stable(using G418 resistance) expression in mammalian cells. Insect cell expression systems, e.g., *Drosophila,* are also useful, see for example, PCT applications WO 90/06358 and WO 92/06212 as well as EP 290,261-B1.

Transcription of DNA by higher eukaryotes is increased by inserting an enhancer sequence into the vector. Enhancers are cis-acting elements of DNA, usually about from 10 to 300 bp that act on a promoter to increase its transcription. Examples including the SV40 enhancer on the late side of the replication origin bp 100 to 270, a cytomegalovirus early promoter enhancer, the polyoma enhancer on the late side of the replication origin, and adenovirus enhancers.

Host cell containing the above vectors can be a higher eukaryotic cell, such as a mammalian cell, or a lower eukaryotic cell, such as a yeast cell, or the host cell can be a prokaryotic cell, such as a bacterial cell. As representative examples of appropriate hosts, there may be mentioned: prokaryotes for example bacterial cells, such as *E. coli, Streptomyces, Salmonella typhimurium* and eukaryotes for example fungal cells, such as yeast, insect cells such as *Drosophila* and *Spodoptera frugiperda*, mammalian cells such as CHO, COS or Bowes melanoma, plant cells, etc. The selection of an appropriate host is deemed to be within the scope of those skilled in the art from the teachings herein.

Introduction of the construct into the host cell can be effected by calcium phosphate transfection, DEAE-Dextran mediated transfection, or electroporation. (Davis, L., Dibner, M., Battey, I., Basic Methods in Molecular Biology, (1986)).

Following transformation of a suitable host strain and growth of the host strain to an appropriate cell density, the selected promoter is induced by appropriate means (e.g., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well know to those skilled in the art.

Various mammalian cell culture systems can also be employed to express recombinant protein. Examples of mammalian expression systems include the COS-7 lines of monkey kidney fibroblasts, described by Gluzman, *Cell*, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

Transgenic non-human animals may also be used as hosts.

Where the assay of the invention is cell free, the method of recovery of expressed polypeptide depends on the expression system and host selected. If the expression system secretes the polypeptide into growth media, the polypeptide can be purified directly from the media. If the polypeptide is not secreted, it is isolated from cell lysates or recovered from the cell membrane fraction. Where the polypeptide is localized to the cell surface, whole cells or isolated membranes can be used as an assayable source of the desired gene product. Polypeptide expressed in bacterial hosts such as *E. coli* may require isolation from inclusion bodies and refolding. The selection of the appropriate growth conditions and recovery methods are within the skill of the art.

The polypeptide can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography hydroxylapatite chromatography and lectin chromatography. Protein refolding steps can be used, as necessary, in completing configuration of the mature protein. Finally, high performance liquid chromatography (HPLC) can be employed for final purification steps.

Depending upon the host employed in a recombinant production procedure, the polypeptides may be glycosylated or may be non-glycosylated. Polypeptides may also include an initial methionine amino acid residue.

"Recombinant" polypeptides refer to polypeptides produced by recombinant DNA techniques; i.e., produced from cells transformed by an exogenous DNA construct encoding the desired polypeptide. "Synthetic" polypeptides are those prepared by chemical synthesis.

A "replicon" is any genetic element (e.g., plasmid, chromosome, virus) that functions as an autonomous unit of DNA replication *in vivo;* i.e., capable of replication under its own control.

A "vector" is a replicon, such as a plasmid, phage, or cosmid, to which another DNA segment may be attached so as to bring about the replication of the attached segment.

A "double-stranded DNA molecule" refers to the polymeric form of deoxyribonucleotides (bases adenine, guanine, thymine, or cytosine) in a double-stranded helix, both relaxed and supercoiled. This term refers only to the primary and secondary structure of the molecule, and does not limit it to any particular tertiary forms. Thus, this term includes double-stranded DNA found, *inter alia*, in linear DNA molecules (e.g., restriction fragments), viruses, plasmids, and chromosomes. In discussing the structure of particular double-stranded DNA molecules, sequences may be described herein according to the normal convention of giving only the sequence in the 5' to 3' direction along the sense strand of DNA.

A DNA "coding sequence of" or a "nucleotide sequence encoding" a particular protein, is a DNA sequence which is transcribed and translated into a polypeptide when placed under the control of appropriate regulatory sequences.

A "promoter sequence" is a DNA regulatory region capable of binding RNA polymerase in a cell and initiating transcription of a downstream (3' direction) coding sequence. Within the promoter sequence will be found a transcription initiation site (conveniently defined by mapping with nuclease S1), as well as protein binding domains (consensus sequences) responsible for the binding of RNA polymerase. Eukaryotic promoters will often, but not always, contain "TATA" boxes and "CAT" boxes.

DNA "control sequences" refers collectively to promoter sequences, ribosome binding sites, polyadenylation signals, transcription termination sequences, upstream regulatory domains, enhancers, and the like, which collectively provide for the expression (i.e., the transcription and translation) of a coding sequence in a host cell.

A control sequence "directs the expression" of a coding sequence in a cell when RNA polymerase will bind the promoter sequence and transcribe the coding sequence into mRNA, which is then translated into the polypeptide encoded by the coding sequence.

A "host cell" is a cell which has been transformed or transfected, or is capable of transformation or transfection by an exogenous DNA sequence.

A cell has been "transformed" by exogenous DNA when such exogenous DNA has been introduced inside the cell membrane. Exogenous DNA may or may not be integrated (covalently linked) into chromosomal DNA making up the genome of the cell. In prokaryotes and yeasts, for example, the exogenous DNA may be maintained on an episomal element, such as a plasmid. With respect to eukaryotic cells, a stably transformed or transfected cell is one in which the exogenous DNA has become integrated into the chromosome so that it is inherited by daughter cells through chromosome replication. This stability is demonstrated by the ability of the eukaryotic cell to establish cell lines or clones comprised of a population of daughter cell containing the exogenous DNA.

A "clone" is a population of cells derived from a single cell or common ancestor by mitosis. A "cell line" is a clone of a primary cell that is capable of stable growth *in vitro* for many generations.

The present invention is also directed to compounds which are inhibitors of Asp 2 modulated APP cleavage, and their use in treating β amyloid protein-related disease including Alzheimer's Disease.

The invention further provides the use of a compound according to the invention in the preparation of a medicament for inhibiting Asp 2 modulated APP cleavage, in particular for the treatment of β amyloid protein-related disease including Alzheimer's Disease.

The invention further provides a method of inhibiting Asp 2 modulated APP cleavage, in particular the treatment or prophylaxis of β amyloid protein-related disease including Alzheimer's Disease, which method comprises administering to a patient an effective amount of a compound of the invention.

When used in therapy, the compounds of the invention are formulated in accordance with standard pharmaceutical practice.

The present invention therefore also provides a pharmaceutical composition comprising a compound of the invention and a pharmaceutically acceptable carrier.

The compounds which are active when given orally can be formulated as liquids, for example syrups, suspensions or emulsions, tablets, capsules and, lozenges.

A liquid formulation will generally consist of a suspension or solution of the compound or pharmaceutically acceptable salt in a suitable liquid carrier(s) for example, ethanol, glycerine, non-aqueous solvent, for example polyethylene glycol, oils, or water with a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

Typical parenteral compositions consist of a solution or suspension of the compound or pharmaceutically acceptable salt in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

A typical suppository formulation comprises a compound of formula (I) or a pharmaceutically acceptable salt thereof which is active when administered in this way, with a binding and/or lubricating agent such as polymeric glycols, gelatins or cocoa butter or other low melting vegetable or synthetic waxes or fats.

Preferably the composition is in unit dose form such as a tablet or capsule.

Each dosage unit for oral administration contains preferably from 1 to 250 mg (and for parenteral administration contains preferably from 0.1 to 25 mg) of an inhibitor of the invention.

The daily dosage regimen for an adult patient may be, for example, an oral dose of between 1 mg and 500 mg, preferably between 1 mg and 250 mg, or an intravenous, subcutaneous, or intramuscular dose of between 0.1 mg and 100 mg, preferably between 0.1 mg and 25 mg, of the compound of the formula (I) or a pharmaceutically acceptable salt thereof calculated as the free base, the compound being administered 1 to 4 times per day. Suitably the compounds will be administered for a period of continuous therapy.

The present invention will be further described with reference to the following examples; however, it is to be understood that the present invention is not limited to such examples. All parts or amounts, unless otherwise specified, are by weight.

### METHODS

### Transient expression of Asp 2 in mammalian cells:

DNA encoding Asp 2 was cloned into an expression vector for transient expression in mammalian cells. The Asp 2 gene was amplified with the primers 5' TATTATCTACTCGAGCCACCATGGCCCAAGCCCTGCC 3' (SEQ ID NO.8) and 5' GTTAATATAAAGCTTCAGCAGGGAGATGTCATCAGC 3' (SEQ ID NO.9) and cloned into pCR2.1 (Invitrogen). The Asp 2 gene was then ligated to pcDNA3.1aMycHis (Invitrogen) digested with *Eco R* I/*Hind* III.

SH-SY5Y human neuroblastoma cells over expressing the human APP isoform 695, (SH-SY5Y APP695) were cultured in Dulbecco's Modified essential medium:F12 supplemented with 10% foetal bovine serum (v/v), penicillin (50 units/ml), streptomycin (50 µg/ml), 2mM glutamine and 260µg/ml hygromycin B at 37 °C in a humidified atmosphere of 10% CO₂/90%air. COS-7 APP751 cells, wild type and with the APP Swedish mutation (Haass, C. *et al*., *Nature. Med.* 1: 1291-1296 (1995)), Lys 595→Asn and Met 596→Leu (APP 695 numbering), were cultured in the above medium but with 300µg/ml hygromycin B. For transient transfection, cells were seeded at ∼60% confluency in 75cm² tissue culture flasks and transfected using LipofectAMINE PLUS Reagent (Life Technologies) as described by the manufacturer. 24hr post transfection the medium was changed to OptiMEM-1 (10ml). The medium was collected 24 hr later, centrifuged briefly (500 x g for 10 min) to remove any cells and then concentrated using Centriprep 10 concentrators (Amicon). Cells were harvested by dissociation from the flasks using an enzyme free cell dissociation buffer (Life Technologies) and lysed by incubation for 30 min at 4°C in 50 mM Tris/HCl pH 7.4, 1% Triton X-100 containing a cocktail of protease inhibitors (Boehringer Mannheim). After centrifugation (3,000 x g for 5 min) the supernatant was aspirated and stored at -20°C until assayed.

### Subcellular localization of Asp 2

A cDNA encoding Asp 2 with a Myc epitope tag was transfected into COS-7 APP-751 cells. Cells were fixed and processed for indirect immunofluorescence as described in Spector, D.L., Goldman, R.D. & Leinwald, L.A. Cells, in a Laboratory Manual, 3, 105.3 , Cold Spring Harbor Lab Press, Cold Spring Harbor, New York (1998). APP was visualised using the anti C-terminal antibody Ab54. Control antibodies were, monoclonal Anti-golgi 58K protein from Sigma, monoclonal Early Endosome Antigen 1 (EEA1) from Transduction Labs, monoclonal Anti KDEL from Stressgen, monoclonal anti Myc from SantaCruz Biotechnology. Detection was with Alexa 488 labelled anti mouse or Alexa 568 labelled anti-rabbit antibodies from Molecular Probes. Microscopy was carried out using a Leica confocal microscope.

### Immunohistochemistry

Asp 2 immunohistochemistry: 10µm sections of paraformaldehyde-fixed hippocampus and frontal and temporal cortex from two Alzheimer's disease patients (female,ages 62 and 89) and two aged controls (female, ages 80 and 86) were rehydrated and labelled with an antibody to Asp 2 by incubation overnight at 4°C. Subsequent processing used the biotin-avidin system (with biotinylated goat anti-rabbit antibody) and the chromogen, diaminobenzidine (Vector Laboratories).

### Mutagenesis:

Asp 2 active site mutants were constructed with the Stratagene QuickChange mutagenesis kit. Two oligonucleotides were designed to introduce each Asp to Asn, 
D93N1:
5' CTCAACATCCTGGTGAATACAGGCAGCAGTAAC 3' (SEQ ID NO.10)
 
D93N2:
5' GTTACTGCTGCCTGTATTCACCAGGATGTTGAG 3' (SEQ ID NO.11)
 
D289N1:
5' GACAAGAGCATTGTGAACAGTGGCACCACCAAC 3'(SEQ ID NO.12)
 
D289N2:
5' GTTGGTGGTGCCACTGTTCACAATGCTCTTGTC 3' (SEQ ID NO.13)
 (positions D93 and D289 referred to above correspond to positions D48 and D244 of the mature protein)
The mutations were introduced into the pcDNA3.1a Asp 2 construct, following the QuickChange protocol. Mutants were sequenced to ensure the presence of the desired active site mutation and absence of PCR mutations.

### SDS PAGE and Western blotting:

Cell lysates (20 µg) or media (15 µl of 20 fold concentrated media) samples were mixed with an equal volume of Lamelli sample buffer (0.5M Tris/HCl, pH 6.8, 10% (w/v) SDS, 0.1% bromophenol blue, 20% (v/v) glycerol and 5% β-mercaptoethanol) and electrophoresed using pre-cast on 10% Tris glycine SDS polyacrylamide gels (Novex). Following electrophoresis, gels were electroblotted onto PVDF at 100V for 60 min using the wet blot apparatus (Novex), the transfer buffer consisted of 35 mM Tris HCl, 193 mM glycine and 20% methanol (v/v) . Following transfer, membranes were blocked in 5% Marvel, phosphate buffered saline (PBS), 0.1% Tween-20 for 3 hr at room temperature. Membranes were then incubated with primary antibody in 2% bovine serum albumin, PBS, 0.1% Tween-20 overnight at 4°C. The anti-His₆ antibody (Boehringer Mannheim) was used at a dilution of 1:1000; the antibody raised to the C-terminus of APP (amino acid sequence 676-695), Ab54 (Allsop, D. *et al*., in *Alzheimer's Disease: Biology, Diagnostics and Therapeutics*, eds Iqbal, K., Winblad, B., Nishimura, T., Takeda, M. & Wisneski, H.M., 717-727 , John Wiley, New York (1997)), was used at a dilution of 1:25000, antibody WO2 (Ida N. *et al., J.Biol.Chem*. 271: 22908-22914 (1996)) that was raised to amino acids 1-16 of the Aβ domain of APP was used at a dilution of 1:3000 and antibody 1A9 raised to the neoepitope region of soluble APP generated after cleavage by β-secretase (Le Brocque, D. et., *Biochem.* 37: 14558-14565 (1998)) was used at a dilution of 1:3000. Bound antibody was detected using a peroxidase conjugated secondary antibody (Sigma) and with an additional peroxidase anti-peroxidase antibody for the membranes probed with antibody 1A9 and antibody WO2, in conjunction with the enhanced chemiluminscence (ECL) detection method (Amersham).

For the APP C-terminal fragments cell lysates (20 µg) were mixed with an equal volume of Lamelli sample buffer (0.5M Tris/HCl, pH 6.8, 10% (w/v) SDS, 0.1% bromophenol blue, 20% (v/v) glycerol and 5% β-mercaptoethanol) and electrophoresed using pre-cast on 10-20% Tris trycine SDS polyacrylamide gels (Novex). Following electrophoresis, gels were electroblotted onto 0.45 µm nitrocellulose at 0.38A for 35 min using the wet blot apparatus (Novex), the transfer buffer consisted of 35 mM Tris HCl, 193 mM glycine, 0.01% SDS and 20% methanol (v/v). Following transfer the blot was microwaved in boiling PBS for 10 min. Following transfer, for detection with Ab54 membranes were blocked in 5% milk powder, phosphate buffered saline (PBS), 0.1% Tween-20 for 1 hr at room temperature. Membranes were then incubated with Ab54 (used at a dilution of 1:25000) in 2% bovine serum albumin, PBS, 0.1% Tween-20 overnight at 4°C. For WO2 the membranes were blocked in 10% milk powder, PBS for 1 hour at room temperature. Membranes were then incubated with WO2 at 1µg/ml in PBS overnight at 4°C. Bound antibody was detected using a peroxidase conjugated secondary antibody (Sigma) in conjunction with the enhanced chemiluminscence (ECL) detection method (Amersham).

### Results

### Demonstration of Asp 2 immunoreactivity in humans

AD and control hippocampus was immunostained for Asp 2 with a protein A purified polyclonal antiserum raised to a peptide sequence derived from Asp 2. There was clear neuronal staining, but there was no staining associated with astrocytes, microglia or oligodendrocytes. While some neurones appeared to be more intensely labelled than others, they all showed a similar staining pattern with the immunoreactivity localising to the cytoplasm of the perikaryon and dendrite only. The dendritic staining rarely extended beyond 10-15µm and no axonal labelling was observed. Within the positive cell bodies themselves, the staining was non-uniform and showed slight granularity. Intraneuronal staining was also evident in frontal and temporal cortex and in brain from aged control subjects.

Asp 2 was found to be present in untransfected SH-SY5Y cells stably expressing the 695 isoform of APP (SH-SY5Y APP-695) and in COS-7 cells expressing the 751 isoform of APP (COS-7 APP-751). The level of Asp 2 was increased upon transient transfection with the vector pcDNA3.1 carrying the Asp 2 gene. Upon transient transfection with the protein Asp 2 was present as a major band at 65kDa. Upon prolonged exposure of the ECL blot a weaker band at 60kDa was evident. Deglycosylation experiments showed that these two bands are differentially glycosylated forms of the same protein.

### Subcellular localisation of Asp 2 and APP

Vector pcDNA3.1 carrying Myc epitope tagged Asp 2 was transfected into COS-7 APP-751 cells. Consistent with several reports (Kuentzel, S.L., Ali, S.M., Altman, R.A., Greenberg, B.D. & Raub, T.J. *J. Biochem.* 295, 367-378 (1993), Walter, J *et al., Mol. Med.* 2, 673-691 (1996)), APP clearly localised to the Golgi/endoplasmic reticulum region as revealed by distinctive juxtanuclear staining and a more generalised reticular staining throughout the cell. Asp 2 showed essentially the same subcellular distribution and clear co-localisation with APP as revealed by simultaneous detection of Myc tagged Asp 2 and APP in COS-7 APP-751 cells. Interestingly the co-localisation is not absolute; APP was detected more readily towards the cell periphery than Asp 2. This suggests that these two proteins may segregate as they are processed and transported within the cell. The distribution of both of these proteins is quite distinct from the distribution of markers that define the early endosome and the endoplasmic reticulum.

### Effect of the expression of Asp 2 and Asp 2 active site mutants on sAPPβ secretion

Both of the active site mutants and Asp 2 were expressed to similar levels in the SH-SY5Y APP-695 cells. An increase in sAPPβ (110kDa) was observed in the media from Asp 2 transfected cells compared to empty vector pcDNA3.1MycHis transfected control cells. There was no increase in sAPPβ secreted from cells transfected with either of the Asp-Asn mutants D93N or D289N. In contrast to that seen with sAPPβ, Asp 2 had no effect on the secretion of soluble APPα or on full length APP in the cell.

### Effect of the expression of Asp 2 and Asp 2 active site mutants on APP C terminal fragments

The C-terminal fragments of APP were detected in COS-7 cells stably expressing APP-751 with and without the Swedish mutation. 12kDa and 10kDa bands were detected by Ab54 raised to the C-terminal region of APP. The 12kDa fragment was immunoreactive with the antibody WO2 which is specific for residues 5-9 of human Aβ and is thus the C-terminal fragment produced by the action of β-secretase (CTFβ). The 10kDa fragment band was not immunoreactive with this antibody, and based upon this and its molecular weight is therefore CTFα, the C-terminal fragment produced by the action of α-secretase. The C-terminal fragment produced by the action of γ-secretase was not detected; this may be due to the low levels of this fragment or its rapid clearance in the cell.

Transfection with Asp 2 resulted in an accumulation of the 12kDa CTF in COS-7 APP-751 cells as detected by the Ab54 and WO2 antibodies. Transient transfection with Asp 2 also caused an accumulation of this 12kDa β-secretase derived CTF in COS-7 expressing APP-751 bearing the Swedish mutation.

There was no increase in CTFβ with the D93N and D289N active site mutants, thus confirming that expression of endocrepsin-2 encoded a functioning proteinase which required the presence of both of the catalytic aspartic residues.

### Preparation of Asp 2 - Fc fusion protein (Asp 2(1-460)-Fc)

A cDNA fragment encoding Asp 2, amino acids 1 to 460 (EP855444 but with 130 Glu->Val), was subcloned to create'a fusion protein with human immunoglobulin, residues 99-330 of IgG1, and cloned into the mammalian expression vector pCDN (Aiyar et al). The plasmid was linearized by digestion with Not 1 (15ug DNA, 37'C, overnight), sterially precipitated and resuspended into 50ul 1X TE buffer (10mM Tris, 1mM EDTA, pH 7.5). The DNA was electroporated, using a Bio-Rad Gene Pulser (Bio-Rad Laboratories) into a chinese hamster ovary (CHO E1A) cell line (derived from DG-44 (Urlaub et al) adapted for growth in suspension in maintance medium) using the technique of Hensley et. al. The cells were plated into 96 well culture plates at 5 X 10⁵ cells/plate in maintance medium for 24 hr prior to selection. Cells were selected in maintance medium without nucleosides (selection medium). Conditioned medium from individual colonies was assayed using an electrochemiluminescence detection method on an Origen analyzer (IGEN) the technology reviewed in Yang et al 1994. A high expressing colony was scaled into 250 ml shake flasks containing 30 liters of selection medium to generate conditioned medium for purification.
Aiyar, N., Baker, E., Wu, H-L, E., Nambi, P., Edwards, R.M., Trill, J.J., Ellis, C., Bergsma, D. Human AT1 receptor is a single copy gene: characterization in a stable cell line. Molecular and Cellular Biochemistry 131:75-86,1994
Urlaub, G., Kas, E., Carothers, A.M., and Chasin, L.A. (1983) Cell 33, 405-412
Hensley, P., McDevitt, P.J., Brooks, I., Trill, J.J., Feild, J.A., McNulty, D.E., Connor, J.R., Griswold, D.E., Kumar, V., Kopple, K.D., Carr, S.A., Dalton, B.J., Johanson, K. The soluble form of E-selectin is an asymmetric monomer: Expression, purification and characterization of the recombinant protein. J. Biol. Chem 269:23949-23958, 1994.
Yang, H., Leland, J.K., Yost, D., Massey, R.J. Electrochemiluminescence: A new diagnostic and research tool. Biotechnology, 12:193-194, 1994

### Asp 2 - Fc Purification:

Asp 2-Fc prepared as above was captured from CHO E1a medium by Protein A affinity chromatography (ProSepA resin from BioProcessing Ltd). The fusion protein was eluted from the column with 0.1 M glycine-HCl, pH 3.0, neutralized with 1 M Tris-HCl, pH 8.0, and dialyzed against 25 mM HEPES pH 7.4 containing 0.25 M NaCl. N-terminal sequencing and SDS-PAGE analysis in the presence and absence of DTT showed that the protein was a disulfide linked heterodimer of Asp2/Fc - Fc. N-Terminal sequencing showed that all of signal sequence was processed, the protein consisting of 37.5 % preform (starting at Thr22) and 62.5 % mature form (starting at Glu46).

### Fluorescence Resonsance Energy Transfer (FRET) Cleavage Assay for Asp2.

A peptide (X) based on the sequence of the Swedish variant APP beta-site sequence has been doubly labelled for use in a FRET assay. 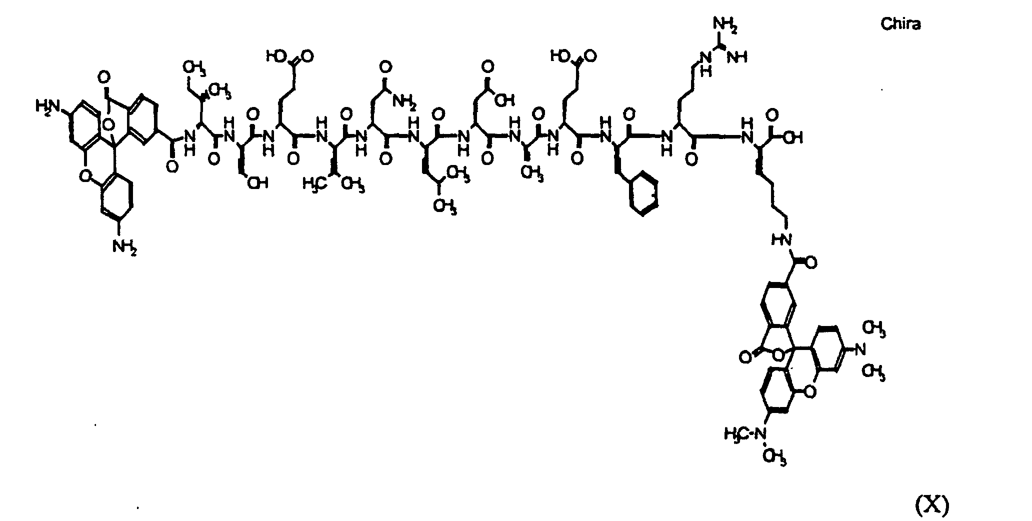 This peptide includes the 11 residues spanning the Swedish variant beta-cleavage site (Ile-Ser-Glu-Val-Asn-Leu*Asp-Ala-Glu-Phe-Arg) with an N-terminal rhodamine green group and C-terminal tetramethylrhodamine group. This would allow cleavage of the intervening peptide sequence by Asp2 to be monitored by energy transfer between the two fluorescent groups.

In practice assays were conducted as follows:
The fluorescent peptide substrate (0.5 uM) was incubated with Asp2-Fc enzyme, prepared as described above, in a buffer containing 50 mM sodium acetate, 20 mM sodium chloride, 5 % glycerol, 0.1 % CHAPS (3-[(3-Cholamidopropyl) dimethylammonio]-1-propane-sulphanate), pH 3.8). The assays were started by addition of enzyme (25 nM final concentration) and cleavage monitored on a fluorescent platereader with 485 nm excitation, 538 nm emission. The C-terminal TAMRA group of the peptide acted as effective quencher, thus an increase in rhodamine green intensity was observed upon cleavage.

This assay was used to determine IC50 and Kᵢ values for compounds of interest. A number of hydroxyethylene compunds were found to be inhibitory in this assay, including: 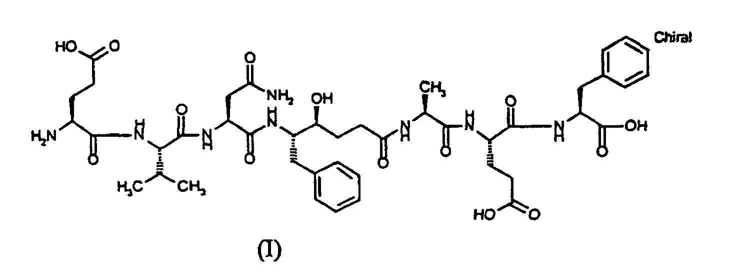 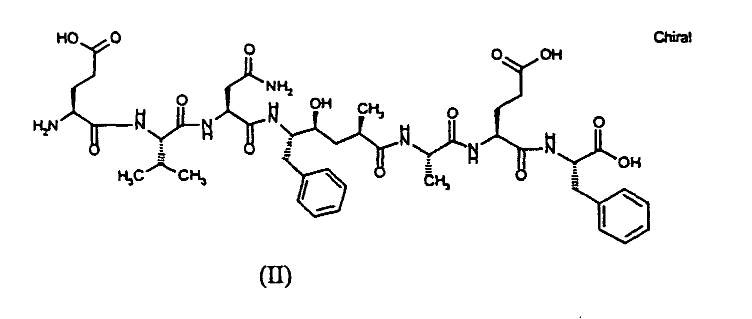 Kᵢ values of 7.7 nM and 7.3 nM were determined for (I) and (II) respectively.

### Asp 2 Fluorescence Polarisation Assay.

A competition assay has also been designed to identify compounds acting at the Asp2 active site. The hydroxyethylene inhibitor, (I), has been modified by addition of 5-, 6-rhodamine green to the free N-terminal amine of the compound to create labelled ligand (III). 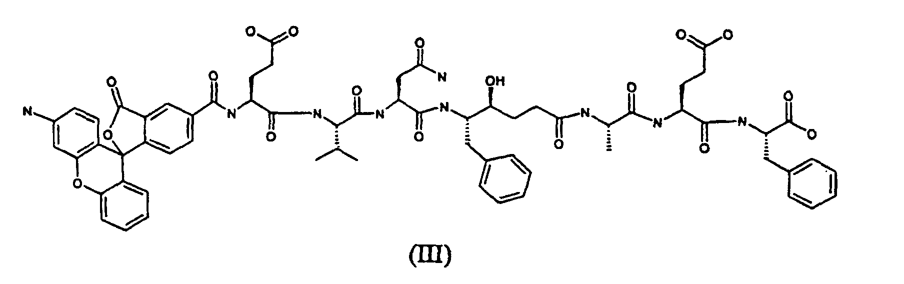 The resulting compound was tested experimentally as follows to determine the K_{d} with Asp2-Fc enzyme, prepared as above.

A three dimensional analysis varying enzyme and compound (III) was performed. Ligand concentration range was 1, 3, 10, 30 and 100 nM. Enzyme concentration range was from 100 nM down in serial twofold dilutions (11 dilutions + no enzyme control). Ligand of appropriate concentration was prepared in 5% DMSO. The enzyme solutions were prepared in 2 times (x2) assay buffer (where x1= 50 mM sodium acetate, 20 mM sodium chloride, 5% glycerol, 0.1% CHAPS, pH 4.0). 5 ul of the enzyme solution (of varying concentration) was mixed with 5 ul of the ligand (of varying concentration) in a low volume 384 microtitre plate. The samples were allowed to equilibrate for 15 min before the fluorescence polarisation was read on an LJL Acquest platereader.

The Anisotropy values were plotted and the K_{d} for the tight binding interaction obtained. A K_{d} of 1.22 nM was obtained for the interaction.

Compound (IV) was tested in the same assay to demonstrate competition against compound (III):.

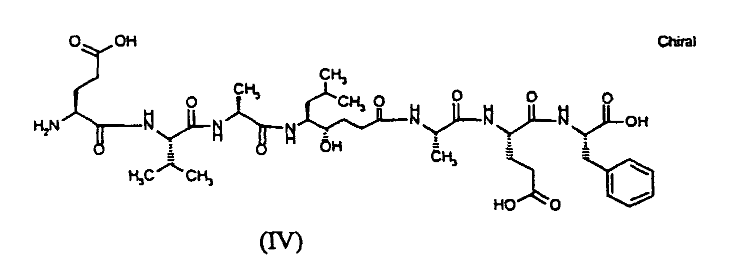

Enzyme (at a fixed concentration of 25 nM), ligand (III) (at a fixed concentration of 2.5 nM) and test compound (10 uM down in twofold serial dilutions) were mixed in x 1 assay buffer (50 mM 50 mM sodium acetate, 20 mM sodium chloride, 5% glycerol, 0.1% CHAPS, pH 3.8) in a final assay volume of 10 ul and allowed to equilibrate for 3 hrs. The fluorescence polarisation was then read. The experiment was performed in duplicate.

Complete displacement of the fluorescent ligand (III) was clearly observed at the highest concentrations of test compound in the assay. An average Kᵢ of 67.5 nM was obtained.

## Claims

1. A method of screening compounds to identify those compounds which inhibit the Asp 2 mediated cleavage of a polypeptide substrate, the method comprising:
providing in a cell free assay a reaction system comprising purified recombinant Asp 2,optionally as an Fc fusion, and substrate in a suitable reaction buffer; and
measuring the extent of cleavage of the substrate in the presence of test compound as compared with the extent of cleavage in the absence of test compound, wherein the peptide substrate contains a pair of marker groups which straddle the cleavage site, such that cleavage of the substrate directly results in the modulation of a fluorescent signal, one marker group carries the signal generator and the other marker group performs the modulator function and wherein the marker groups are xanthenes selected from rhodamines, rhodols and fluoresceins, whereby the absorption spectrum of the modulator (acceptor) overlaps the emission spectrum of the generator (donor) such that changes in energy transfer are observed upon cleavage of the peptide.

2. A method according to claim 1 wherein the generator/modulator pair is selected from rhodamine/rhodamine and fluorescein/rhodamine.

3. A method according to claim 2 wherein the generator/modulator pair is rhodamine green/tetramethylyrhodamine.

4. A method according to claim 3 wherein the substrate is of formula (X):

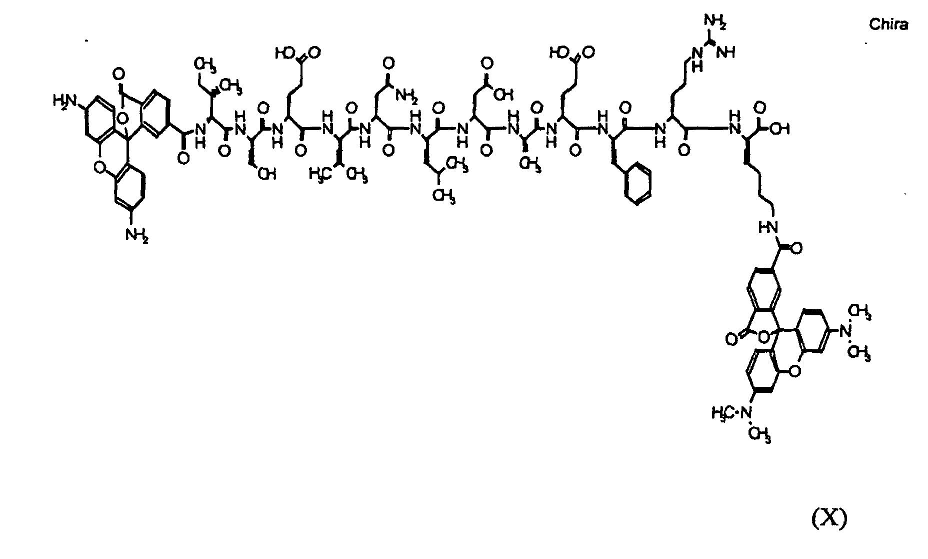

5. A method according to claim 2 wherein the generator/modulator pair is 5-(and/or 6)-carboxyfluorescein and 5-(and/or 6)-tetramethylrhodamine (TAMRA).

6. A method according to claim 1 wherein the substrate is a peptide spanning the beta-secretase cleavage site of APP Swedish variant sequence (from P6 to P5').

7. A method according to claim 2 wherein the substrate is:

Ile-Ser-Glu-Val-Asn-Leu-Asp-Ala-Glu-Phe-Arg

8. A method of screening compounds to identify those compounds which inhibit the Asp 2 mediated cleavage of a polypeptide or protein substrate, the method comprising: providing a reaction system comprising Asp 2 and labelled active site ligand; and measuring the extent of binding of labelled ligand in the presence of test compound as compared with the extent of binding of labelled ligand in the absence of test compound in order to determine binding affinity, wherein the assay method for the ligand is based on translational or rotational diffusion.

9. A method according to claim 8 wherein the assay method is based on fluorescence polarisation (FP).

10. A method according to claim 9 wherein the labelled ligand is a compound of formula (III):

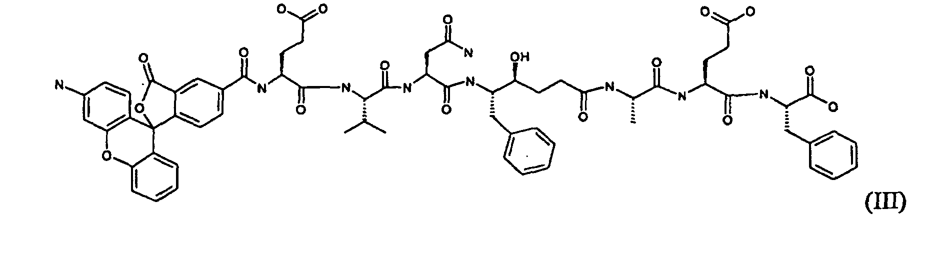

## Patentansprüche

1. Verfahren zum Screenen von Verbindungen, um jene Verbindungen zu identifizieren, die die durch Asp 2 vermittelte Spaltung eines Polypeptidsubstrats hemmen, wobei das Verfahren umfasst:
Bereitstellen eines Reaktionssystems in einem zellfreien Assay, wobei das Reaktionssystem gereinigtes rekombinantes Asp 2, gegebenenfalls als ein Fc-Fusionsprotein, und Substrat in einem geeigneten Reaktionspuffer umfasst;
und Messen des Ausmaßes der Spaltung des Substrats in der Gegenwart einer Testverbindung im Vergleich mit dem Ausmaß der Spaltung in Abwesenheit der Testverbindung, wobei das Peptidsubstrat ein Paar von Markergruppen enthält, die beiderseits der Spaltstelle liegen, sodass die Spaltung des Substrats unmittelbar zur Modulation eines Fluoreszenzsignals führt, eine Markergruppe den Signalerzeuger trägt und die andere Markergruppe die Modulatorfunktion ausübt, und wobei die Markergruppen Xanthene sind, ausgewählt aus Rhodaminen, Rhodolen und Fluoresceinen, wobei das Absorptionsspektrum des Modulators (Akzeptors) mit dem Emissionsspektrum des Erzeugers (Donors) überlappt, sodass Änderungen des Energietransfers nach Spaltung des Peptids beobachtet werden.

2. Verfahren nach Anspruch 1, wobei das Erzeuger-/Modulatorpaar ausgewählt ist aus Rhodamin/Rhodamin und Fluorescein/Rhodamin.

3. Verfahren nach Anspruch 2, wobei das Erzeuger-/Modulatorpaar Rhodamin Green/Tetramethylrhodamin ist.

4. Verfahren nach Anspruch 3, wobei das Substrat die Formel (X) hat:

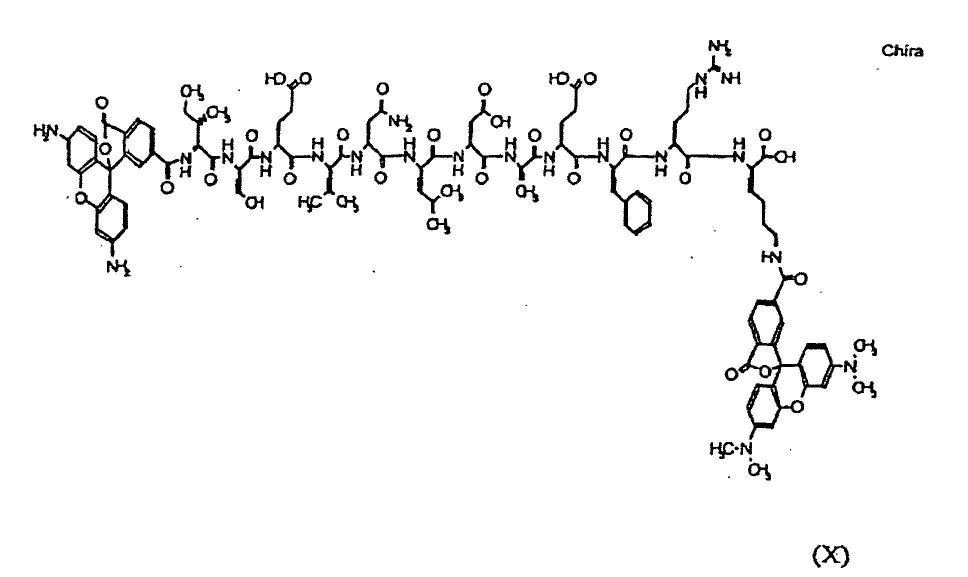

5. Verfahren nach Anspruch 2, wobei das Erzeuger-/Modulatorpaar 5-(und/oder 6)-Carboxyfluorescein und 5-(und/oder 6)-Tetramethylrhodamin (TAMRA) ist.

6. Verfahren nach Anspruch 1, wobei das Substrat ein Peptid ist, das die Beta-Secretase-Spaltstelle der schwedischen Variante der APP-Sequenz (von P6 bis P5') überspannt.

7. Verfahren nach Anspruch 2, wobei das Substrat Ile-Ser-Glu-Val-Asn-Leu-Asp-Ala-Glu-Phe-Arg ist.

8. Verfahren zum Screenen von Verbindungen, um jene Verbindungen zu identifizieren, die die durch Asp 2 vermittelte Spaltung eines Polypeptid- oder Proteinsubstrats hemmen, wobei das Verfahren umfasst: Bereitstellen eines Reaktionssystems, das Asp 2 und einen markierten Ligand des aktiven Zentrums umfasst; und Messen des Ausmaßes des Bindens des markierten Liganden in Gegenwart einer Testverbindung im Vergleich mit dem Ausmaß des Bindens des markierten Liganden in Abwesenheit der Testverbindung, um die Bindungsaffinität zu bestimmen, wobei das Assayverfahren für den Liganden auf Translations- oder Rotationsdiffusion basiert.

9. Verfahren nach Anspruch 8, wobei das Assayverfahren auf Fluoreszenzpolarisation (FP) basiert.

10. Verfahren nach Anspruch 9, wobei der markierte Ligand eine Verbindung mit der Formel (III) ist:

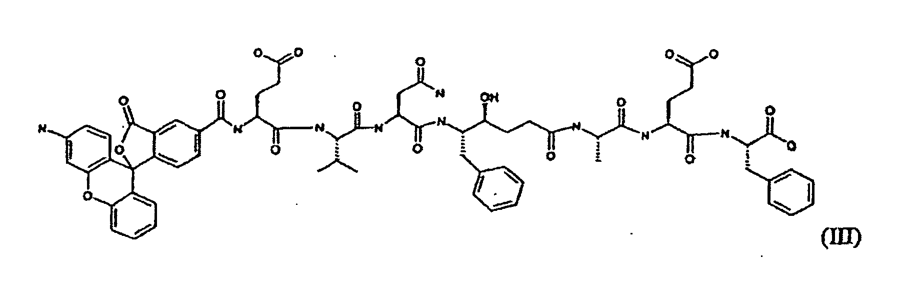

## Revendications

1. Procédé pour sélectionner des composés afin d'identifier les composés qui inhibent le clivage, à médiation par Asp 2, d'un substrat polypeptidique, procédé comprenant les étapes consistant : à fournir, dans une analyse acellulaire, un système réactionnel comprenant de la Asp 2 recombinante purifiée, éventuellement sous forme d'une fusion de Fc, et un substrat dans un tampon réactionnel convenable ; et à mesurer le degré de clivage du substrat en présence d'un composé d'essai comparativement au degré de clivage en l'absence du composé d'essai, dans lequel le substrat peptidique contient une paire de groupes servant de marqueurs qui chevauchent le site de clivage, de telle sorte que le clivage du substrat a directement pour résultat la modulation d'un signal fluorescent, un groupe servant de marqueur portant le générateur de signal et l'autre groupe servant de marqueur assurant la fonction de modulateur, et dans lequel les groupes servant de marqueurs sont des xanthènes choisis entre des rhodamines, des rhodols et des fluorescéines, le. spectre d'absorption du modulateur (accepteur) chevauchant le spectre d'émission du générateur (donneur) de telle sorte que des variations de transfert d'énergie sont observées lors du clivage du peptide.

2. Procédé suivant la revendication 1, dans lequel la paire générateur/modulateur est choisie entre la paire rhodamine/rhodamine et la paire fluorescéine/rhodamine.

3. Procédé suivant la revendication 2, dans lequel la paire générateur/modulateur est la paire vert de rhodamine/tétraméthylrhodamine.

4. Procédé suivant la revendication 3, dans lequel le substrat répond à la formule (X) :

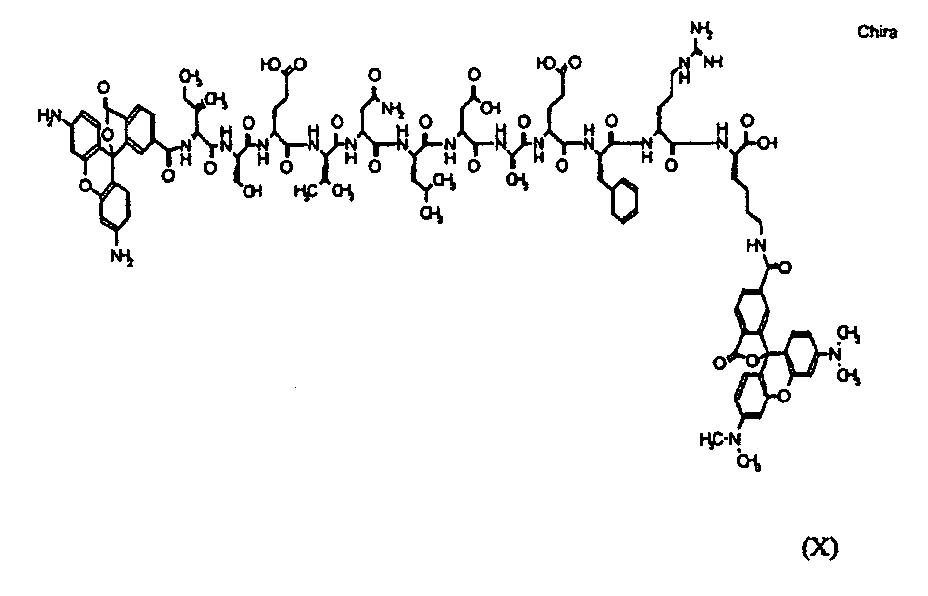

5. Procédé suivant la revendication 2, dans lequel la paire générateur/modulateur est constituée de 5-(et/ou 6)-carboxyfluorescéine et de 5-(et/ou 6)-tétraméthylrhodamine (TAMRA).

6. Procédé suivant la revendication 1, dans lequel le substrat est un peptide s'étendant sur le site de clivage par la bêta-sécrétase de la séquence de variant suédois de APP (de P6 à P5').

7. Procédé suivant la revendication 2, dans lequel le substrat est :

Ile-Ser-Glu-Val-Asn-Leu-Asp-Ala-Glu-Phe-Arg

8. Procédé pour sélectionner des composés afin d'identifier les composés qui inhibent le clivage, à médiation par Asp 2, d'un substrat polypeptidique ou protéique, ledit procédé comprenant les étapes consistant : à fournir un système réactionnel comprenant Asp 2 et un ligand marqué de site actif ; et à mesurer le degré de liaison du ligand marqué en présence d'un composé d'essai comparativement au degré de liaison du ligand marqué en l'absence du composé d'essai afin de déterminer l'affinité de liaison, la méthode d'analyse du ligand étant basée sur la diffusion par translation ou rotation.

9. Procédé suivant la revendication 8, dans lequel la méthode d'analyse est basée sur la polarisation de fluorescence (FP).

10. Procédé suivant la revendication 9, dans lequel le ligand marqué est un composé de formule (III) :
